Europäisches Patentamt

European Patent Office

Office européen des brevets

(19)

(11) Publication number: 0 369 714
A1

## (12) EUROPEAN PATENT APPLICATION

(21) Application number: 89311732.5

(22) Date of filing: 13.11.89

(51) Int. Cl.⁵: **A61K 33/24**

(30) Priority: **14.11.88 US 270330**

(43) Date of publication of application:
**23.05.90 Bulletin 90/21**

(84) Designated Contracting States:
**AT BE CH DE ES FR GB GR IT LI LU NL SE**

(71) Applicant: **BRISTOL-MYERS SQUIBB
COMPANY**
**345 Park Avenue**
**New York, N.Y. 10154(US)**

(72) Inventor: **Kaplan, Murray A.**
**1026 Glencove Road**
**Syracuse New York(US)**
Inventor: **Perrone, Robert K.**
**7353 Tomwood Drive**
**Liverpool New York(US)**
Inventor: **Bogardus, Joseph B.**
**8239 Penstock Way**
**Manlius New York(US)**
Inventor: **Douglas, Kenneth W., Sr.**
**P.O. Box 841**
**Mexico New York 13114(US)**

(74) Representative: **Jones, Alan John et al**
**CARPMAELS & RANSFORD 43 Bloomsbury
Square**
**London, WC1A 2RA(GB)**

(54) Cisplatin hypertonic solution.

(57) Disclosed herein are stable, ready-to-use cisplatin aqueous solutions containing high concentrations of chloride ion. Solutions of this invention contain less aquated platinum species and are closer to physiological pH than known cisplatin solutions. Also included are lyophilized cisplatin compositions having high chloride concentrations.

EP 0 369 714 A1

# CISPLATIN HYPERTONIC SOLUTION

## BACKGROUND OF THE INVENTION

### 1. Field of the Invention

The present invention relates to pharmaceutical compositions of cisplatin.

### 2. Background Art

Cisplatin has emerged as an important antineoplastic agent and is currently approved in the United States for the treatment of metastatic testicular cancer, metastatic ovarian cancer, and advanced bladder cancer.

Cisplatin is commonly available as a lyophilized powder which is reconstituted with Water for Injection, U.S.P. before use. For example, the Division of Cancer Treatment, National Cancer Institute provides investigators lyophilized cisplatin which upon reconstitution with water yields a solution containing for each ml, 1 mg of cisplatin, 9 mg of sodium chloride, and 10 mg of mannitol; the pH of the resulting solution is about 3.5 to 5.5 (NCI Investigational Drugs, Pharmaceutical Data, 1985, pp 74-77). US Patent 4,670,262 discloses a freeze-dried cisplatin composition containing a lower alcohol. Ready-to-use cisplatin solutions have been disclosed in US Patent 4,310,515 and European Application 143,478. A recent Japanese Kokai 62/292,722 (Derwent No. 88-032992) discloses a solution of cisplatin in physiological saline and containing magnesium chloride to lower nephrotoxicity. However, due to the inherent instability of cisplatin in aqueous environment, the reconstituted lyophilized cisplatin or the ready-to-use cisplatin solutions show formation of considerable amounts of "aquated platinum species". These degradation products have been implicated as possible agents for cisplatin toxicities and therefore it would be desirable to have formulations containing as little as possible of these species.

Greene et al (Am. J. Hosp. Pharmacy, 1979, 36:38-43), using NCI cisplatin formulations, showed that in less than one hour after reconstitution with 0.9% sodium chloride solution at room temperature approximately 3% of cisplatin was degraded and that level remained constant for 24 hours; even greater cisplatin loss was observed at lower sodium chloride concentrations. The pH of the cisplatin saline solutions in the Greene study was approximately 4. Hincal et al (J. Parenteral Drug Assoc., 1979, 33:107-16) reported the stabilizing effect of up to 0.9% sodium chloride on cisplatin aqueous solutions over 24 hours. In the same study cisplatin was found to be unstable in 5% sodium bicarbonate solution of pH 7.5 at 25°C, and it was suggested that alkaline solution of cisplatin should be avoided since the équilibria may be unfavorably shifted at higher pH.

Litterst (Toxicol. Appl. Pharmacol., 1981, 61:99-108) studied the toxicity in mice of cisplatin administered in a hypertonic solution containing 4.5% sodium chloride. The cisplatin used was obtained from the Division of Cancer Treatment, National Cancer Institute. The study showed that cisplatin administered in high salt solution is significantly less toxic than the drug administered in water or normal saline.

Aamdal et al (J. Natl. Cancer Inst., 1984, 73:743-752) also prepared cisplatin in hypertonic saline solution using commercially available formulations containing in addition to cisplatin, mannitol and sodium chloride. Solutions containing 1 mg/ml cisplatin and 1.8%, 4.0%, and 6.0% (w/v) sodium chloride were prepared and the effect of high sodium chloride concentrations on antitumor activity of cisplatin was evaluated in mice. The lyophilized cisplatin used was obtained from Farmitalia Carlo Erba (Milan, Italy) and Bristol Laboratories (Syracuse, NY).

Ozols et al (Ann. Internal Med., 1984, 100:19-24) administered 40 mg/m$^2$ cisplatin to humans as a 30 minute infusion in 250 ml 3% saline. Assuming a body surface area of 2 m$^2$, the solution administered would have a cisplatin concentration of 0.32 mg/ml.

In the three studies cited above, the pH of the solutions was not adjusted and the stability of the solutions was not evaluated. We have determined the pH of one vial of cisplatin reconstituted with 5% sodium chloride and it was 4.75. This is consistent with the pH range given in the NCI monograph. It is expected that reconstitution with 0.9% sodium chloride would yield a solution with similar pH as concentration of salt appears to have little effect on pH.

# EP 0 369 714 A1

## SUMMARY OF THE INVENTION

The present invention provides a stable ready-to-use cisplatin aqueous solution containing for each ml up to 1 mg of cisplatin, and a pharmaceutically acceptable source of chloride ion in amount equivalent to that produced by about 20 to 100 mg of sodium chloride; the pH of the solution may range from about 5.0 to about 7.5.

A further aspect of the invention provides a cisplatin pharmaceutical dosage form for reconstitution with parenteral diluent which comprises for each part of cisplatin, 25 to 100 parts of a pharmaceutically acceptable source of chloride ion, said dosage form being substantially free of lower alkanol.

## DETAILED DESCRIPTION OF THE INVENTION

Cisplatin is known to be unstable in aqueous solutions and rapidly establishes equilibrium with the formation of various "aquated platinum species", dimers, and trimers (Fairlie and Whitehouse, Biochem. Pharmacol., 31, 933 (1982)). The formation of these decomposition products represents not only a loss of cisplatin activity but also an increase of substances potentially more toxic than cisplatin. Although studies have shown cisplatin in 0.9% sodium chloride solution decomposes to a lesser extent than in solutions with lower sodium chloride concentration, the loss of cisplatin is still approximately 3% in 24 hours at room temperature. Furthermore, because the equilibrium is unfavorably shifted to degradation products under alkaline conditions, heretofore available ready-to-use solutions all have pH values ranging from about 2 to about 4. Such acidic injectable solutions may be highly irritating and thus an added burden to patients undergoing chemotherapy.

It has been discovered that the above objections may be overcome by supplying to aqueous cisplatin solutions a high concentration of chloride ion thereby enabling the preparation of stable ready-to-use cisplatin solutions that are closer to physiological pH than those currently available.

The ready-to-use cisplatin solution of the present invention contains up to 1 mg/ml of cisplatin, and a pharmaceutically acceptable source of chloride ion in amount equivalent to that produced by about 20 to about 100 mg/ml of sodium chloride. The pH of the solution is adjusted to about 5.0 - 7.5. Preferably, the cisplatin concentration is from about 0.2 to about 1.0 mg/ml. The preferred source of chloride ion is sodium chloride which is preferably present in concentration of about 40 to about 60 mg/ml with about 50 mg/ml being the most preferred. We found that although sodium chloride may be present in concentration higher than 60 mg/ml, any higher salt concentrations do not confer significant additional stability to the ready-to-use cisplatin solutions. A preferred pH range for the solution is from about 5.6 to about 6.5, and most preferably from about 6.0 to about 6.5. The cisplatin solution of the present invention may be used in substantially the same manner as known ready-to-use cisplatin solutions.

The solution of the present invention is readily prepared by dissolving cisplatin in the desired concentration in water containing sodium chloride. It is preferred to dissolve the sodium chloride first in the water and then add cisplatin, because of the above-mentioned formation of aquated species. Although sodium chloride is the preferred source of chloride ion, other pharmaceutically acceptable sources of chloride ion, such as potassium chloride, calcium chloride, N-methylglucamine hydrochloride, choline chloride, and the like may also be used. If necessary, the pH of the solution is adjusted to the desired level by addition of a pharmaceutically acceptable acid such as hydrochloric acid or a pharmaceutically acceptable base such as sodium hydroxide. The solution may be either substantially free of other ingredients or it may contain other pharmaceutical excipients such as mannitol. Preservatives may also be added if desired. The bulk cisplatin should be protected from light; therefore, the preparative process is preferably carried out under reduced light exposure, either in the dark or in highly-subdued light.

Instruction for the preparation of a cisplatin solution containing 1 mg/ml cisplatin and 50 mg/ml sodium chloride is provided as follows:

1. Place approximately 900 ml of water for injection, U.S.P. at 20 - 30°C in a suitable container.
2. Add and dissolve 50 g of sodium chloride with stirring.
3. In the dark (or in highly-subdued light), cautiously sprinkle in 1.0 g of cisplatin over a 5-minute interval and with rapid stirring. Stir until dissolved (approximately 2 - 4 hours is required).
4. Add 0.1 - 1.0 N NaOH to adjust pH to about 5.5 - 6.5 Stir for 0.5 hour.
5. Bring the volume to 1 L with Water for Injection, U.S.P. Stir in the dark for an additional 1 hour.
6. Using aseptic techniques throughout and protecting the solution from light:

3

a) Pass the solution through a suitable 0.22 micron membrane filter.

b) Collect the filtrate in a suitable container.

c) Fill required volumes into suitable brown-glass vials. Seal with suitable stoppers (20 mm Daikyo) and aluminum closures.

Accordingly, a cisplatin solution containing 1 mg/ml cisplatin, 50 mg/ml sodium chloride and having a pH of 6.5 was prepared. Similarly, by substituting 0.5 g for the 1.0 g of cisplatin and 25 g for the 50 g of sodium chloride previously used, a solution containing 0.5 mg/ml cisplatin and 25 mg sodium chloride was obtained.

Cisplatin solutions containing about 0.6 mg/ml or higher of cisplatin may deposit cisplatin crystals if kept at 4°C. The crystals, however, may be re-dissolved by shaking and warming to 40°C. Solutions containing 0.5 mg/ml or less of cisplatin do not deposit cisplatin crystals when stored at 4°C.

The stability of solutions containing 1 mg/ml cisplatin, either 50 mg/ml or 9 mg/ml of sodium chloride and having a pH of 2.5, 4.2 and 6.5 was evaluated at 25°, 37°, and 45°. Samples were periodically assayed for cisplatin, trichloroamminoplatinum (TCAP), and aquated species, using high performance liquid chromatography. The results are presented in Table I.

Table I

| | | | Months 25°C | | | | | Months 37°C | | | | Weeks 45°C | | | |
|---|---|---|---|---|---|---|---|---|---|---|---|---|---|---|---|
| Degradation of 1 mg/ml cisplatin solution | | | | | | | | | | | | | | | |
| | | Original | 2 | 3 | 4 | 6 | 12 | 1 | 2 | 3 | 4 | 1 | 2 | 4 | 8 |
| **pH 2.5** | | | | | | | | | | | | | | | |
| 0.9%NaCl | % cisplatin Rem. | | 98.5 | 99.0 | 100.7 | 97.2 | 98.0 | 95.4 | 96.7 | 96.8 | 96.4 | 98.2 | 96.6 | 95.5 | 97.1 |
| | % TCAP | 0 | 0 | 0.5 | 0.2 | 0.45 | 1.3 | 0.8 | 1.1 | 2.4 | 2.0 | 0.18 | 0.44 | 0.8 | 1.4 |
| | % Aquated sp. | 2.2 | 1.2 | 3.0 | 4.4 | 3.2 | 2.6 | 1.6 | 2.2 | 2.9 | 1.8 | 0.8 | 5.4 | 1.0 | 2.4 |
| 5%NaCl | % cisplatin Rem. | | 102.1 | 99.7 | 102.0 | 99.7 | 100.6 | 97.3 | 99.2 | 96.1 | 96.9 | 99.7 | 99.2 | 96.2 | 97.6 |
| | % TCAP | 0 | 0.1 | 0.68 | .39 | 0.58 | 1.4 | 1.1 | 2.7 | 6.2 | 5.1 | 0.29 | 0.58 | 1.7 | 3.7 |
| | % Aquated sp. | 0 | 0 | 0 | 0 | 0 | 0 | 0 | 0 | 0 | 0 | 0 | 0 | 0 | 0 |
| **pH 4.2** | | | | | | | | | | | | | | | |
| 0.9%NaCl | % cisplatin Rem. | | 98.7 | 99.5 | 101.8 | 97.3 | 99.0 | 95.8 | 97.5 | 97.3 | 97.2 | 99.0 | 94.6 | 92.8 | 96.3 |
| | % TCAP | 0 | 0 | 0.27 | 0.2 | 0.45 | 0.8 | 0.6 | 0.6 | 2.0 | 1.4 | 0.1 | 0.3 | 0.55 | 1.5 |
| | % Aquated sp. | 2.0 | 1.3 | 3.0 | 1.2 | 3.2 | 2.0 | 1.0 | 1.3 | 2.9 | 3.7 | 1.6 | 1.5 | 3.0 | 3.0 |
| 5%NaCl | % cisplatin Rem. | | 100.9 | 99.8 | 102.1 | 97.9 | 100.6 | 95.5 | 98.2 | 96.0 | 97.1 | 99.2 | 99.5 | 94.4 | 97.7 |
| | % TCAP | 0 | 0.19 | 0.58 | 0.29 | 0.29 | 0.87 | 0.68 | 1.9 | 4.1 | 2.8 | 0.19 | 0.44 | 1.4 | 2.7 |
| | % Aquated sp. | 0 | 0 | 0 | 0 | 1.4 | 0 | 0 | 0 | 1.6 | 0.8 | 0 | 0.5 | 0 | 1.4 |
| **pH 6.5** | | | | | | | | | | | | | | | |
| 0.9%NaCl | % cisplatin Rem. | | 94.5 | 95.5 | 96.5 | 90.7 | 93.6 | 89.9 | 91.5 | 91.5 | 90.3 | 93.3 | 88.3 | 89.5 | 89.9 |
| | % TCAP | 0 | 0 | 0.46 | 0.1 | 0.19 | 0.67 | 0.48 | 0.86 | 2.6 | 1.1 | 0.1 | 0.1 | 0.57 | 1.2 |
| | % Aquated sp. | 6.4 | 3.3 | 4.0 | 4.7 | 9.1 | 3.0 | 5.6 | 6.1 | 3.8 | 7.9 | 4.4 | 8.1 | 5.1 | 6.0 |
| 5%NaCl | % cisplatin Rem. | | 100.8 | 100.1 | 100.9 | 97.2 | 100.3 | 94.7 | 97.2 | 95.8 | 96.7 | 100.5 | 99.8 | 94.0 | 96.7 |
| | % TCAP | 0 | 0 | 0.39 | 0.58 | 0.29 | 0.58 | 0.48 | 2.1 | 4.8 | 7.0 | 0.15 | 0.39 | 1.1 | 2.8 |
| | % Aquated sp. | 0 | 1.0 | 0 | 1.8 | 3.2 | 0.6 | 4.1 | 2.8 | 2.0 | 2.0 | 0.7 | 0.6 | 2.9 | 3.3 |

It is evident from the data in Table I that solutions containing 5% sodium chloride contain significantly less of aquated species than solutions containing 0.9% sodium chloride at the same pH. Although 5% sodium chloride solutions show slightly elevated levels of trichloroamminoplatinum (TCAP), this is considered to be an acceptable compromise because TCAP is known to be far less acutely toxic than cisplatin or the aquated species. It is also evident from Table I that with 5% sodium chloride, the amount of intact cisplatin at pH 6.5 is comparable to that at pH 2.5 and pH 4.2 throughout the duration of the experiment. This is in sharp contrast to the 0.9% sodium chloride solution in which cisplatin is extensively degraded at pH 6.5 compared to pH 2.5 and 4.2. Thus, the ready-to-use cisplatin solutions of the present invention show similar stability profile to known ready-to-use solutions but without the disadvantage of high solution acidity.

The cisplatin solutions as described above may be lyophilized. The lyophilized product comprises for each part of cisplatin, 25 to 100 parts of sodium chloride. A pharmaceutically acceptable filler, for example, mannitol may be optionally included in about 10 to 150 parts relative to cisplatin. The amount of sodium chloride should be sufficient to generate a solution having sodium chloride concentration of at least 45 mg/ml upon reconstitution with water. For example, a vial of the lyophilized dosage form may contain from 5 to 10 mg of cisplatin, 225 to 500 mg of sodium chloride and 50 to 500 mg of mannitol. The pH of the solution for lyophilization may be adjusted to a pH range of about 2 - 3 using hydrochloric acid; however, pH adjustment is not necessary. For clinical usage, the lyophilized cisplatin is first reconstituted with a conventional parenteral diluent such as Water for Injection, U.S.P. to yield a solution containing 1 mg/ml of cisplatin.

The following is a typical procedure for preparing lyophilized cisplatin of the present invention.

1. In highly subdued indirect light for all the following and at 20 - 25°C, place approximately 900 ml of Water for Injection, U.S.P. in a suitable calibrated container.

2. Add, with rapid stirring, 50.0 g of sodium chloride and 10.0 g of mannitol. Stir until the ingredients are dissolved.

3. Sprinkle in 1.0 g of cisplatin over a 5 minute interval. Stir until the cisplatin is dissolved. Stir an additional 10 minutes.

4. Stop stirring and bring the volume to 1 liter with Water for Injection, U.S.P.

5. Stir vigorously for an additional 0.5 hour.

Using aseptic procedures:

6. Pass the solution under nitrogen pressure through a 0.2 micron pore size membrane filter and collect the filtrate.

7. Fill 10 ml of the solution into 20 ml amber flint glass vials.

8. Lyophilize at 0°F for 72 hours and then at 80°F for 48 hours.

9. Insert 20 mm Daikyo stoppers and seal with aluminum closures.

According to the above procedure, each vial contains 10 mg of cisplatin, 500 mg of sodium chloride, and 100 mg of mannitol.

The solid state stability of lyophilized cisplatin and the stability of reconstituted solutions were evaluated using four formulations A, B, C, and D, having the following compositions and characteristics:

| Formulation | A | B | C | D |
|---|---|---|---|---|
| Cisplatin (mg/vial) | 5 | 5 | 5 | 5 |
| NaCl (mg/vial) | 250 | 250 | 45 | 45 |
| Mannitol (mg/vial) | 50 | 50 | 50 | 50 |
| HCl[1] | - | + | - | + |
| pH (reconst. to ~1 mg/ml) | 6.45 | 3.38 | 6.05 | 3.35 |
| Cisplatin potency | | | | |
| assayed directly | 5.000 | 4.925 | 4.920 | 5.140 |
| assayed with added Cl[2] | 5.030 | 4.870 | 5.040 | 5.205 |
| % change[3] | +0.6 | -1.1 | +2.4 | +1.3 |

[1] + = pH of pre-lyophilized solution was adjusted to 2.5 using 1N HCl; - = pH not adjusted (pH = 4.7-5.0).

[2] assayed following a two-fold dilution with pH 2.5 (HCl) 10% NaCl diluent.

[3] general indicator of amount of aquated platinum species present.

Table II

| Stability of lyophilized cisplatin solid[1] | | | | |
|---|---|---|---|---|
| | % of original cisplatin remaining | | | |
| Formulation | 45° | | 56° C | |
| | 2 wk | 4 wk | 2 wk | 4 wk |
| A | 99.8(0)[2] | 100.5(0) | 99.1(0) | 100.0(0) |
| B | 102.7(0) | 98.7(.4) | 99.6(0) | 98.1(.3) |
| C | 97.3 (.69)[3] | 97.4 (.8)[3] | 97.8 (.6)[3] | 96.6 (.79)[3] |
| D | 97.8 (.57)[3] | 98.0 (.58)[3] | 97.0 (.77)[3] | 97.4 (.86)[3] |

[1] Reconstituted with Water for Injection, U.S.P. at the Time of assay.

[2] % TCAP shown in parenthesis.

[3] Original cisplatin potency is that determined with a two-fold dilution with pH 2.5 (HCl), 10% NaCl diluent.

Table III

| Stability of cisplatin reconstituted to 1 mg/ml and diluted with parenteral vehicles (24° C) | | | | |
|---|---|---|---|---|
| | % cisplatin remaining | | | |
| | Diluted 10-fold | | Diluted 10-fold | |
| Formulation | with 5% Dextrose | | with 0.9% NaCl | |
| | 4 hr | 24 hr | 4 hr | 24 hr |
| A | 94.5 | 94.8 | 95.3 | 94.3 |
| B | 95.6 | 92.9 | 96.9 | 95.5 |
| C | 83.7 | 79.6 | 94.7 | 93.1 |
| D | 82.7 | 79.6 | 98.5 | 95.0 |

Table II shows that the examples of the present invention (formulations A and B) are more stable than the prior art systems (C and D) at extreme temperatures. Such compositions would be useful in tropical countries or other climates where storage of the product at 20 - 25°C cannot be assured. The generally lower potencies of formulations C and D are due in part to the formation of aquated species as discussed previously.

The data in Table III indicate that the use of 5% NaCl in cisplatin lyophile allows dilution of the reconstituted solution into diverse diluents, including chloride-absent vehicles such as 5% dextrose. The percent cisplatin remaining at 4 and 24 hours show that dilution of reconstituted lyophile containing 0.9% NaCl (Formulation C and D) in 5% dextrose would be unacceptable due to loss in potency and the formation of very large amounts of toxic aquated species. As also shown in Table III, a 10-fold dilution of the reconstituted lyophile containing 5% NaCl using normal saline (0.9% NaCl) has equal potency to that of similarly diluted reconstituted lyophile containing 0.9% NaCl. This is due to a similar chloride concentration of about 1% NaCl in the final solutions.

It is noteworthy that all cisplatin reference standard solutions used for stability work were prepared freshly as 1 mg/ml cisplatin in 5% NaCl (w/v), 0.05 N HCl solutions. This assures minimal formation of "aquated Pt species" in the standard. Cisplatin solutions prepared in water or normal saline would be lower in cisplatin potency due to rapid equilibration with "aquated-Pt-species", thus, assays using these standards may produce artificially high cisplatin potency.

It is to be understood that the scope of the invention is not limited by the compositions and parameters specifically exemplified to illustrate the invention, but is defined by the following claims.

## Claims

1. A stable aqueous solution of cisplatin in a sealed container suitable for parenteral administration comprising for each milliliter of solution up to 1.0 milligram of cisplatin and a pharmaceutically acceptable source of chloride ion in an amount equivalent to that produced by about 20 to 100 milligrams of sodium chloride, said solution having a pH of from about 5.0 to about 7.5.

2. A solution of Claim 1 wherein said chloride source is sodium chloride.

3. A solution of Claim 1 or Claim 2 wherein the pH of said solution is from about 5.6 to about 6.5.

4. A solution of Claim 1 or Claim 2 wherein the pH of said solution is from about 6.0 to about 6.5.

5. A solution of any one of Claims 1 to 4 wherein for each milliliter of solutions the amount of chloride ion is equivalent to that produced by 40 to 60 milligrams of sodium chloride.

6. A solution of Claim 2 containing for each milliliter of solution about 40 to 60 milligrams of sodium chloride, said solution having a pH of from about 5.6 to about 6.5.

7. A solution of Claim 6 wherein the pH of said solution is from about 6.0 to about 6.5.

8. A solution of Claim 2 containing for each milliliter of solution about 0.2 to 1.0 milligram of cisplatin and about 45 to 55 milligrams of sodium chloride, said solution having a pH of from about 5.6 to about 6.5.

9. A solution of Claim 8 wherein the pH of said solution is from about 6.0 to about 6.5.

10. A solution of Claim 8 containing for each milliliter of solution about 0.5 to 1.0 milligram of cisplatin and about 50 milligrams of sodium chloride, said solution having a pH of from about 6.0 to about 6.5.

11. A solution of Claim 10 wherein the pH of said solution is about 6.5.

12. A pharmaceutical dosage form of cisplatin for reconstitution with parenteral diluent which comprises for each part of cisplatin about 25 to 100 parts of sodium chloride, said dosage form being substantially free of lower alkanol.

13. A dosage form of Claim 12 which further comprises about 10 to 150 parts of a pharmaceutically acceptable filler.

14. A dosage form of Claim 13 wherein said filler is mannitol.

15. A process for preparing a ready to use cisplatin solution which comprises the steps of

(a) dissolving sodium chloride in water to form a saline solution containing about 20 to about 100 mg of sodium chloride per ml of solution;

(b) adding cisplatin to the solution of (a), and agitating to form a solution containing up to 1 mg of cisplatin per ml of solution; and

(c) adjusting the pH of the solution of (b) to about 5.0 to about 7.5.

16. A process for preparing a cisplatin dosage form which comprises the steps of

(a) dissolving sodium chloride and a pharmaceutically acceptable filler in water to form a saline solution containing about 20 to about 100 mg of sodium chloride and about 10 to about 150 mg of the filler per ml of solution;

(b) adding cisplatin to the solution of (a), and agitating to form a solution containing up to 1 mg of cisplatin per ml of solution; and

(c) lyophilizing the solution of (b).

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | Dictionnaire Vidal 1985, O.V.P. PARIS, FR, Page 290; "Cisplatyl" <br> * Page 290: "Cisplatyl" * | 1,2,12, 13,14 | A 61 K 33/24 |
| X | Dictionnaire Vidal 1985, O.V.P. Pars, FR, page 290; "Cisplatine Lilly" <br> * Page 290: "Cisplatine Lilly" * | 1,2,12, 13,14 | |
| X | CHEMICAL ABSTRACTS, vol. 95, 1981, page 25, abstract no. 197173z, Columbus, Ohio, US; C.L. LITTERST et al.: "Alterations in the toxicity of cis-dichlorodiammineplatinum-II and in tissue localization of platinum as a function of sodium chloride concentration in the vehicle of administration", & TOXICOL. APPL. PhARMACOL. 1981, 61(1), 99-108 <br> * Abstract * | 1,2,12 | |
| X | GB-A-2 074 028 (BRISTOL-MYERS) <br> * Claims 1,2,3,7 * | 1,2,12 | TECHNICAL FIELDS SEARCHED (Int. Cl.5) <br><br> A 61 K |

The present search report has been drawn up for all claims

| Place of search | Date of completion of the search | Examiner |
|---|---|---|
| THE HAGUE | 19-02-1990 | PEETERS J.C. |